# EUROPEAN PATENT APPLICATION

(11) **EP 4 138 092 A1**
(43) Date of publication of application: **22.02.2023**
(21) Application number: 21191441.1
(22) Date of filing: 16.08.2021
(51) Int. Cl.: G16H 40/63, A61B 6/00

(54) **DETECTION OF IMPROPERLY CONFIGURED MEDICAL IMAGING SYSTEMS**

(71) Applicant: Koninklijke Philips N.V., 5656 AG Eindhoven (NL)
(72) Inventor: LIPS, Oliver, 5656 AE Eindhoven (NL); NIELSEN, Tim, 5656 AE Eindhoven (NL)
(74) Representative: Philips Intellectual Property & Standards

(57) **Abstract**

Disclosed herein is a medical system (100, 300) comprising a memory (120) storing machine executable instructions (120) and a trained outlier detection module (122). The trained outlier detection module is configured to output an identification of one or more inconsistent data elements (128) in response to inputting configuration data (124) and sensor data (126) from a tomographic medical imaging system (302) as input. Execution of the machine executable instructions causes a computational system (104) to: receive (200) the configuration data; receive (202) the sensor data from the tomographic medical imaging system; input (204) the configuration data and the sensor data into the trained outlier detection module; and provide (206) a signal (130) descriptive of the one or more inconsistent data elements if the identification of the one or more inconsistent data elements is received from the trained outlier detection module.

## Description

### FIELD OF THE INVENTION

The invention relates to medical imaging, in particular to the configuration of a medical imaging system.

### BACKGROUND OF THE INVENTION

Various tomographic medical imaging techniques such as Magnetic Resonance Imaging (MRI), Computed Tomography, Positron Emission Tomography, and Single Photon Emission Tomography enable detailed visualization of anatomical structure of a subject. A common feature of all of these imaging modalities is that the imaging system needs to be configured.

Unites States patent application publication US20200124691A1 discloses imaging protocols are modified in real time through implementation of artificial intelligence. Key steps include: inputting of medical images and patient data; performing artificial intelligence analysis; outputting a potentially modified radiological imaging examination protocol; if applicable, an option for the radiologist to review the patient data, images acquired and the Al's potentially modified radiological imaging examination protocol; delivery of the modified radiological imaging examination protocol to the imaging device; and, if applicable, an option for the radiologist to provide feedback and re-train the artificial intelligence algorithm. Further, some of the images that are utilized by the AI system include processed images, such as segmented and filtered volume rendered images.

### SUMMARY OF THE INVENTION

The invention provides for a medical system, a computer program, and a method in the independent claims. Embodiments are given in the dependent claims.

A challenge in using tomographic medical imaging systems, such a magnetic resonance imaging system, is that the configuration of the system can be complicated. In a clinical setting, operators will often times enter incorrect configuration and metadata descriptive of the subject. This may have consequences for the image quality, the safety of the subject as well as cause problems with properly analyzing the resulting medical images afterwards. Embodiments may provide for an improved configuration of a tomographic medical imaging system by using a trained outlier detection module to check both configuration data and sensor data received from the tomographic medical imaging system. This may help to ensure that not only did the operator configure the tomographic medical imaging system correctly but also to ensure that the tomographic medical imaging system is functioning properly.

In one aspect the invention provides for a medical system that comprises a memory storing machine-executable instructions and a trained outlier detection module. The machine-executable instructions are for execution by a computational system. The trained outlier detection module may be executable by the computational system or remove / external to the computational system (for example as a cloud based service.). The trained outlier detection module is configured to output an identification of one or more inconsistent data elements in response to inputting configuration data and sensor data from a tomographic medical imaging system as input. An outlier in the sensor data as use herein is an observation or measurement that does not fit or is inconsistent with the rest of a dataset.

The one or more inconsistent data elements may be selected from the configuration data and/or the sensor data. If the configuration data is identified as being an outlier, then some of the configuration data may be data that is input or in error or is incorrect. If the sensor data indicates that it is inconsistent with the rest of the dataset it may indicate that there is some sort of hardware fault in the tomographic medical imaging system.

Tomography as used herein is an imaging that uses sections or sectioning for the use of any kind of penetrating wave. This for example includes magnetic resonance imaging, computed tomography, single photon emission tomography, positron emission tomography and various other imaging techniques that are related to magnetic resonance imaging such as electrical impedance tomography.

The configuration data is descriptive of a configuration of the tomographic medical imaging system to acquire tomographic medical imaging data of a subject. The configuration data as used herein encompasses data used to configure and/or modify a magnetic resonance imaging scan or acquisition. The configuration data may encompass such things as a calibration, calibration measurements, or calibration data used to configure a magnetic resonance imaging system. Concrete examples include shim currents or the resonance frequency in the imaging zone. The configuration data may also encompass user configuration data or data which is entered or altered by a user or operator to configure the magnetic resonance imaging system. Concrete examples off user configuration data include metadata descriptive of the subject, the position of the subject, the orientation of the subject, the scan geometry of the subject, and/or modifications made to the pulse sequence commands.

The tomographic medical imaging data as used herein is the measurements recorded or measured by the tomographic medical imaging system on the subject. These may of course be reconstructed into a medical image.

The medical system further comprises a computational system. Execution of the machine-executable instructions causes the computational system to receive the configuration data. Execution of the machine-executable instructions further causes the computational system to receive the sensor data from the tomographic medical imaging system. Execution of the machine-executable instructions further causes the computational system to input the configuration data and the sensor data into the trained outlier detection module.

Execution of the machine-executable instructions further causes the computational system to provide a signal descriptive of the one or more inconsistent data elements if the identification of the one or more inconsistent data elements is received from the trained outlier detection module. This embodiment may be beneficial because the signal may be used to indicate a fault and provide a warning for an operator of the medical system as well as being used as a means to control or improve the operation of the medical system.

In another embodiment execution of the machine-executable instructions further causes the computational system to receive corrected data in response to providing the signal. The corrected data may be data which may be used to replace at least a portion of the one or more inconsistent data elements. Execution of the machine-executable instructions further causes the computational system to provide modified configuration data by modifying the configuration data using the corrected data. For example, if the corrected data is a direct replacement for the inconsistent data elements these inconsistent data elements can be replaced directly with the corrected data. This embodiment may be beneficial because it may provide for a means of correcting the configuration data and may provide for improved functionality and better imaging quality when using the medical system.

The corrected data may be received from several different places. In some examples it may be entered into a user interface or received from a user interface. In other examples an automated algorithm such as an expert system or lookup table may be used to provide the corrected data. In other examples a trained artificial intelligence module may be used to provide the corrected data.

In another embodiment the medical system further comprises a tomographic medical imaging system. Execution of the machine-executable instructions further causes the computational system to control the tomographic medical imaging system with the control commands to acquire the tomographic medical imaging data. Execution of the machine-executable instructions further causes the computational system to reconstruct a medical image from the tomographic medical imaging data. Execution of the machine-executable instructions further causes the computational system to write the medical image, the modified configuration data, and the sensor data to a medical image file. This embodiment may be beneficial because the modified configuration data has been paired with the medical image. This may assist in using the modified user information with the medical image data. For example, it may assist in training other artificial intelligence units.

In another embodiment Execution of the machine-executable instructions further causes the computational system to write at least a portion of the signal to the medical image file. This may be beneficial because the signal is descriptive of the one or more inconsistent data elements. This may be useful in identifying false or inconsistencies in the medical image file.

In another embodiment Execution of the machine-executable instructions further causes the computational system to modify the control commands with the modified configuration data. This embodiment may be particularly beneficial because it then forms a closed control loop and the configuration of the tomographic medical imaging system is therefore improved.

In another embodiment the tomographic medical imaging system is a magnetic resonance imaging system.

In another embodiment the tomographic medical imaging system is a computed tomography system.

In another embodiment the tomographic medical imaging system is a single photon emission tomography system.

In another embodiment the tomographic medical imaging system is a positron emission tomography system.

In another embodiment the tomographic medical imaging system comprises a magnetic resonance imaging system. The memory further contains a weight estimation module configured to output an estimated subject weight in response to inputting the configuration data and the sensor data. In some examples the weight estimation module is identical with the trained artificial intelligence module as described below. The configuration data comprises a user entered subject weight. Execution of the machine-executable instructions further causes the processor to receive the estimated subject weight by inputting the configuration data and the sensor data into the weight estimation module if the identification of the one or more inconsistent data elements comprises the user entered subject weight. Execution of the machine-executable instructions further causes the processor to receive a replacement subject weight selected from one of the estimated subject weight and the user entered subject weight.

This embodiment may be beneficial in improving the safety of the medical system. Often times, when magnetic resonance imaging systems are used, the operator will enter an incorrect weight either because the operator did not properly weight the subject or the subject provided an incorrect weight. Various sensor data may assist in determining if the weight is correct. For example, the position that the subject support is put into as well as looking at the impedance matching and matching network positions for the radio frequency coils of the radio frequency system of the magnetic resonance imaging system.

The weight estimation module could for example be implemented as an expert system. In another example the weight estimation module could be implemented as a fully connected neural network. In this case the sensor data as well as the configuration data may be used to produce a relatively accurate estimate of the subject's weight. This could for example be trained by making weight measurements of subjects and then recording the configuration data and the sensor data. The configuration data and the sensor data can be used as an input into the fully connected neural network and the measured subject weight can be used as the output of the fully connected neural network. Standard deep learning training can be used to train the fully connected neural network. Sets of data which contain correct configuration data, sensor data, and subject weights can be collected and then used for some deep learning to train the fully connected neural network.

In another embodiment the control commands are pulse sequence commands to control the magnetic resonance imaging system to acquire k-space data from an imaging zone at least partially containing the subject. The pulse sequence commands comprise a radio frequency power level setting. Execution of the machine-executable instructions further causes the computational system to calculate the radio frequency power level setting using the replacement subject weight. This embodiment is beneficial because the replacement subject weight can be used to set the radio frequency power level used to acquire the k-space data. This may greatly improve the safety of the magnetic resonance imaging system.

In another embodiment the corrected data is received from a user interface. This may be beneficial because it may provide a means for an operator to respond to the signal. It may also provide a means for the operator to adjust the function of the medical system before the tomographic medical imaging data is acquired.

In another embodiment the user interface is configured to display one or more suggested values for the corrected data. For example, when the signal is provided this may cause a user interface or pop-up box to be displayed on a display to the user. The user interface can then provide a means with suggested values, which may be used to correct the one or more inconsistent data elements. This for example may eliminate the need for the operator to think about what the values should be. This may assist in forming a closed control loop for the medical system.

In another embodiment the corrected data is received from a trained artificial intelligence module. This for example could be implemented using a variety of different means. For example, a fully connected neural network may be used. The trained artificial intelligence module in this case may be trained using deep learning. A training dataset can be collected. For example, the datasets where a signal is provided may be collected over a period of time and an operator can manually enter the correct one or more suggested values. The data was collected with the signal and the manually entered data then can be used to perform the deep learning.

In another embodiment the trained outlier detection module is a fully connected neural network or a random forest regression module. In both ways the training can be achieved in several different ways. In one case the data generated by a medical system can be collected and it can be manually corrected and made into a training dataset. In other cases, the data that is stored with k-space data and other imaging data such as a DICOM image can be mined for the training data.

In another embodiment the configuration data comprises data used to configure and/or modify a magnetic resonance imaging scan or acquisition.

In another embodiment the configuration data comprises calibration data.

In another embodiment the configuration data comprises shim currents.

In another embodiment the configuration data comprises the resonance frequency in the imaging zone.

In another embodiment the configuration data comprises metadata descriptive of the subject.

In another embodiment the configuration data comprises the position of the subject.

In another embodiment the configuration data comprises the orientation of the subject. In another embodiment the configuration data comprises the scan geometry of the subject.

In another embodiment the configuration data comprises modifications made to the pulse sequence commands.

In another embodiment the configuration data comprises a user entered subject weight, an anatomy to be imaged.

In another embodiment the configuration data comprises a logfile of the medical system.

In another embodiment the configuration data comprises error messages of the medical system.

In another embodiment the configuration data comprises B0 shim settings.

In another embodiment the configuration data comprises B1 shim settings.

In another embodiment the sensor data comprises a position of a subject support.

In another embodiment the sensor data comprises a measurement of the ratios of reflected powers in a magnetic resonance imaging system.

In another embodiment the sensor data comprises an impedance network position.

In another embodiment the sensor data comprises a magnetic resonance coil impedance measurement.

In another embodiment the sensor data comprises an X-ray tube voltage measurement. In another embodiment the sensor data comprises an X-ray tube current measurement.

In another aspect the invention provides for a method of operating the medical system. The method comprises receiving configuration data. The configuration data is descriptive of a configuration of a tomographic medical imaging system to acquire tomographic medical imaging data of the subject. The method further comprises receiving the sensor data from the tomographic medical imaging system. The method further comprises inputting the configuration data and the sensor data into a trained outlier detection module. The trained outlier detection module is configured to output an identification of one or more inconsistent data elements in response to inputting configuration data and sensor data as input. The method further comprises providing a signal descriptive of the one or more inconsistent data elements if the identification of the one or more inconsistent data elements is received from the trained outlier detection module.

In another aspect the invention provides for a computer program comprising machine-executable instructions and a trained outlier detection module. The machine-executable instructions and the trained outlier detection module may for example be stored on a non-transitory storage medium. The machine-executable instructions and the trained outlier detection module are configured for execution by a computational system. The trained outlier detection module is configured to output an identification of one or more inconsistent data elements in response to inputting the configuration data and sensor data from a tomographic medical imaging system as input. The configuration data is descriptive of a configuration of the tomographic medical imaging system to acquire tomographic medical imaging data of a subject.

Execution of the machine-executable instructions causes the computational system to receive the configuration data. Execution of the machine-executable instructions further causes the computational system to receive the sensor data from the tomographic medical imaging system. Execution of the machine-executable instructions further causes the computational system to input the configuration data and sensor data into the trained outlier detection module. Execution of the machine-executable instructions further causes the computational system to provide a signal descriptive of the one or more inconsistent data elements if the identification of the one or more inconsistent data elements is received from the trained outlier detection module.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Tomographic medical imaging data (or medical imaging data) is defined herein as being recorded measurements made by a tomographic medical imaging system descriptive of a subject. The tomographic medical imaging data may be reconstructed into a medical image. A medical image is defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the k-space data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a further example of a medical system;
Fig. 4 shows a flow chart which illustrates a method of using the medical system of Fig. 1; and
Fig. 5 illustrates an example of a user interface.

### DESCRIPTION OF EMBODIMENTS

Like numbered elements in these figures are either equivalent elements or perform the same function. Elements which have been discussed previously will not necessarily be discussed in later figures if the function is equivalent.

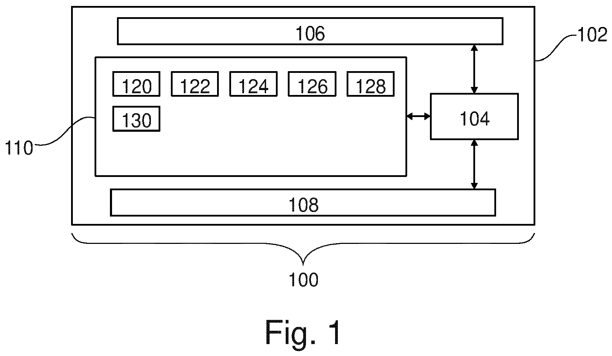

Fig. 1 illustrates an example of a medical system 100 that is shown as comprising a computer 102. The computer 102 comprises a computational system 104. The representation of the computer 102 and the computational system 104 is intended to represent one or more computers 102 or computational systems 104. Various computational tasks could be distributed, for example in the cloud or remotely over the internet.

The computational system 104 is shown as being in communication with an optional hardware interface 106, an optional user interface 108, and a memory 110. The hardware interface 106 enables the computational system 104 to control and communicate with other components. The user interface 108 may enable an operator to control and interact with the medical system 100. The memory 110 is intended to represent various types of memory which may be accessible to the computational system 104.

The memory 110 is shown as containing machine-executable instructions 120. The machine-executable instructions 120 contain instructions that enable the computational system 104 to perform various tasks like data analysis, computational functions, and the control of other components of the medical system 100. The memory 110 is further shown as containing a trained outlier detection module 122. The trained outlier detection module 122 could for example be implemented as a fully connected neural network or a random forest regression model or module. The trained outlier detection module 122 is configured to receive configuration data and sensor data. The configuration data is descriptive of a configuration of a tomographic medical imaging system that is configured to acquire tomographic medical imaging data of a subject. The sensor data is data received from sensors of a tomographic medical imaging system.

The sensor data 126 could be from various components, for example determining the position of a subject support as well as the status of various components. For example, in a magnetic resonance imaging system the sensor data 126 may contain various information such as the type of transceiver or radio frequency coils plugged into the system as well as various impedance and radio frequency measurements made on these coils. The trained outlier detection module 122 is configured to identify and output one or more inconsistent data elements 128 in response to receiving the configuration data and the sensor data. The one or more inconsistent data elements 128 may be chosen from the configuration data 124 and the sensor data 126. The trained outlier detection module 122 detects if the data in the configuration data 124 and the sensor data 126 are self-consistent. If one or more portions of the data 124 or 126 are inconsistent with the others then they are identified in the one or more inconsistent data elements 128. This may for example enable a misconfiguration of the medical system 100 as well as hardware faults to be detected.

The memory 110 is shown as containing the configuration data 124 and sensor data 126 which can be input into the trained outlier detection module 122. The memory 110 is further shown as containing one or more inconsistent data elements 128 that were identified by the trained outlier detection module 122. If the trained outlier detection module 122 does not detect inconsistent data elements 128 it may also output a null result to indicate that no inconsistent data elements were detected. The memory 110 is further shown as containing a signal 130 that was generated in response to the trained outlier detection module 122 outputting the one or more inconsistent data elements 128. The signal may be descriptive of the one or more inconsistent data elements 128.

The signal 130 could in some instances be a warning which is provided to an operator. In other instances, the signal 130 may be used to control algorithms which adjust the operation of the medical system 100. The signal 130 may therefore in some instances be used as part of the control system.

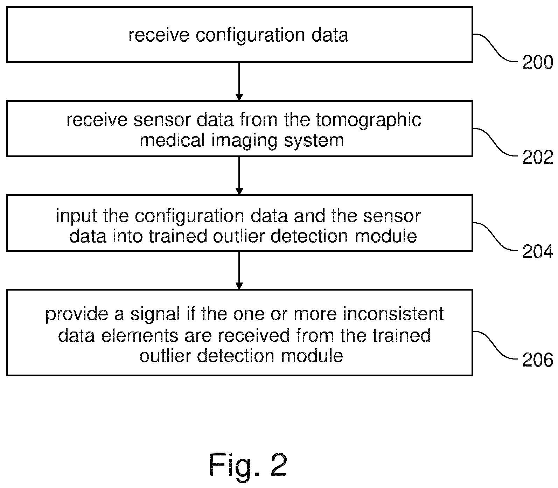

Fig. 2 shows a flowchart which illustrates a method of operating the medical system 100 of Fig. 1. First, in step 200, the configuration data 124 is received. Next, in step 204, the sensor data 126 is received from the tomographic medical imaging system. Then, in step 204, the configuration data 124 and the sensor data 126 are input into the trained outlier detection module 122. If the trained outlier detection module 122 detects any outliers or inconsistent data elements from the sensor data and/or the configuration data 124 then it outputs one or more inconsistent data elements 128. Finally, in step 206, a signal 130 is provided if the one or more inconsistent data elements 128 are received or detected by the trained outlier detection module 122.

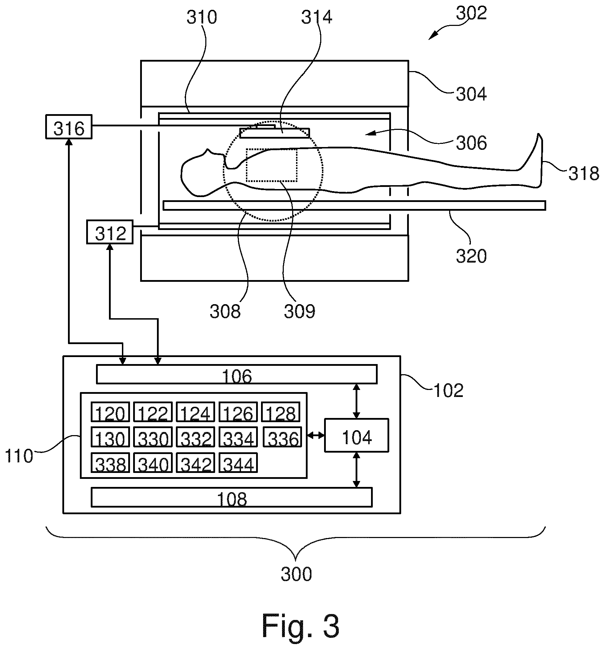

Fig. 3 illustrates a further example of a medical system 300. The medical system 300 depicted in Fig. 3 is similar to the medical system 100 depicted in Fig. 1, except that it additionally comprises a magnetic resonance imaging system that is controlled by the computational system 104. The example illustrated in Fig. 3 is a magnetic resonance imaging system 302. However, the magnetic resonance imaging system could be replaced with other types of tomographic medical imaging systems. An example could be a computed tomography system, a single photon emission tomography system, a positron emission tomography system or a combination of these types of systems.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The magnetic resonance data that is acquired typically acquried for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels.

The transceiver 316 and the gradient controller 312 are shown as being connected to the hardware interface 106 of the computer system 102. Both of these components, as well as others such as the subject support supplying positional data, may supply the sensor data 126.

The memory is shown as containing pulse sequence commands 330. The pulse sequence commands 330 are instructions or data which may be converted into instructions which the computational system 104 can use to control the magnetic resonance imaging system 302 to acquire k-space data 340 from the field of view 309. The pulse sequence commands are an example of control commands. The memory 110 is shown as containing an optional weight estimation module 332. If the one or more inconsistent data elements comprises an user entered subject weight, then the weight estimation module 332 is able to output an estimated subject weight. In this case the estimated subject weight is corrected data 334. The corrected data 334 may for example be provided by different sources. It could be provided by the weight estimation module 332 or it could also be received from a user interface.

The corrected data 334 is used to correct the configuration 124 to provide modified configuration data 336. For example, the data elements in the corrected data 334 may be used to replace a portion of the configuration data 124. In this example the modified configuration data 336 is used to construct modified pulse sequence commands 338. These are an example of modified control commands. If the weight is changed then the modification made to the pulse sequence commands 338 may need to adjust the power delivered by the transceiver 316 to the radio frequency coil. The memory is further shown as containing k-space data 340 that was acquired by controlling the magnetic resonance imaging system 302 with the modified pulse sequence commands 338. The memory is further shown as containing a magnetic resonance image 342 that was reconstructed from the k-space data 340. The memory is further shown as containing an image file 334 which may for example be a DICOM file. The magnetic resonance image 342 as well as at least a portion of the modified configuration data 336 and the sensor data 126 are stored in it.

In the context of a magnetic resonance imaging system 302 the sensor data may take several forms. In one example the sensor data is a position of the subject support 320. This may for example provide a good estimation of the size of the subject 318. It may also be used for detecting faults in the subject support 320. Another item which is maybe very useful as sensor data may be the load placed on the radio frequency coil 314. The amount of mass of the subject 318 will affect the impedance and thereby the load of the radio frequency coil 314. The configuration data 124 in this example may also take several forms. The size of the field of view 309, a shift in the size of the field of view, an indication of the subject position such as being prone or supine, the indication if the subject is head first or feet first in the magnetic resonance imaging system 302 is also useful. A measurement of the ratios of reflected powers is also useful sensor data.

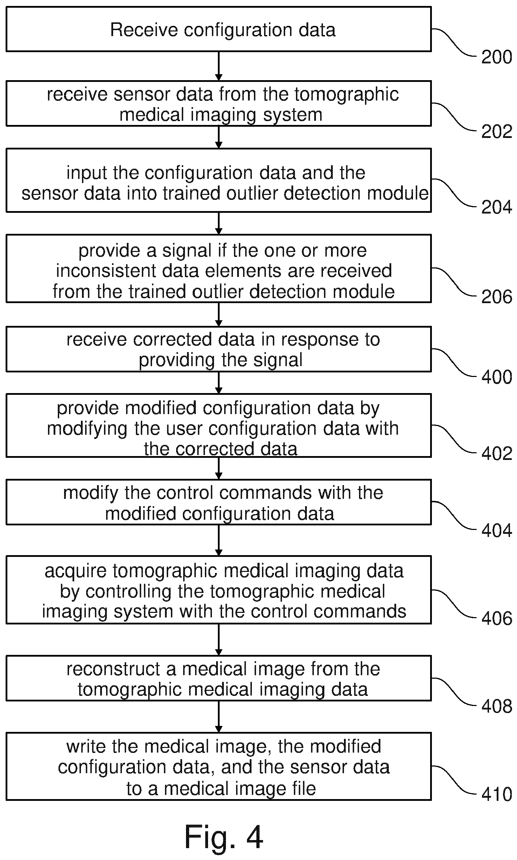

Fig. 4 shows a flowchart which illustrates a method of operating the medical system 300 of Fig. 3. The method illustrated in Fig. 4 is similar to the method illustrated in Fig. 2. Steps 200, 202, 204, and 206, as illustrated in Fig. 2, are performed first in the method illustrated in Fig. 4. After step 206 is performed, in step 400 the corrected data 334 is received in response to providing the signal 130. The corrected data may be received from several different sources. As was mentioned previously, a trained artificial intelligence module or weight estimation module 332 can be used.

The corrected data 334 may also be received from a user interface. Next, in step 402, the modified configuration data 336 is provided by modifying the configuration data 336 with the corrected data 334. In one instance this will mean replacing portions of the configuration data 124 with the data elements contained in the corrected data 334. Next, in step 404, the modified pulse sequence commands 338 or modified control commands are provided by modifying the pulse sequence commands 330 or control commands with the modified configuration data 336. Next, in step 406, the tomographic medical imaging data or k-space data 340 in this particular example, is acquired by controlling the tomographic medical imaging system or magnetic resonance imaging system 302 with the modified control commands or modified pulse sequence commands 338. Next, in step 408, the medical image, or in this particular example the magnetic resonance image 342, is reconstructed from the tomographic medical imaging data or k-space data. Finally, in step 410, the medical image 342, the modified configuration data and the sensor data are written to a medical image file 344.

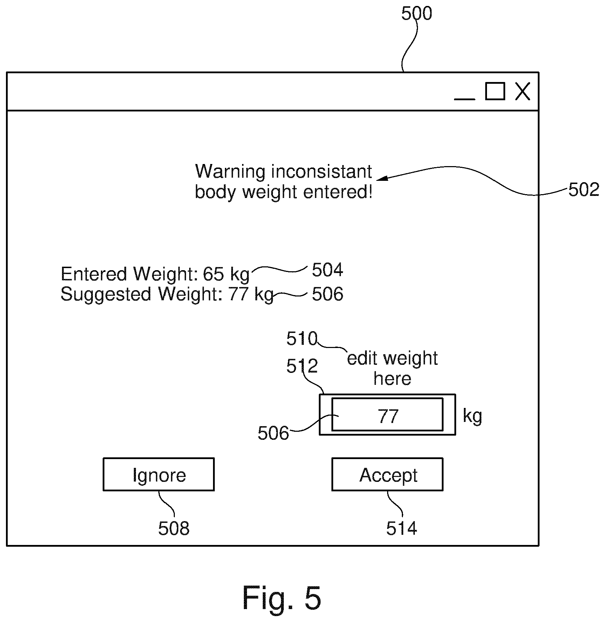

Fig. 5 illustrates an example of a user interface 500 that can be used to provide the corrected data 334. The user interface 500 contains an optional warning 502. In this case it indicates that the body weight of the subject is inconsistent. Also displayed optionally is the entered weight of 65 kg 504 that is the user entered subject weight 504. An estimated subject weight 506 is also displayed and is 77 kg. This is significantly higher and may be due to the subject providing an incorrect or estimate of his or her weight.

The user interface 500 is shown as containing an ignore control button 508 that enables a user to ignore the warning and proceed without changing the weight. Displayed on the user interface 500 are optional instructions 510, which direct the user to modify the user entered subject weight 504 and provide the corrected data 334. In this instance the machine-executable instructions have already entered the estimated subject weight 506 into the data entry box 512. The operator has the option of replacing the value in the data entry box 512. Once the correct weight is in the data entry box 512 the operator can press the accept control button 514 and the contents of the data entry box are provided as the corrected data 334. The corrected data 334 is then the replacement subject weight.

Data is becoming more and more important in modern medical imaging - not only for diagnosis, but also for operational excellence as well as for maintenance and service. In this context, it is beneficial to have data (configuration data 124 and/or sensor data 126) without inconsistencies and flaws. Some of these inconsistencies are due to incorrect user input, e.g. in specifying the anatomy to be imaged or the patient position/orientation. It is proposed to cross-check the data relating to user input by an AI model (trained outlier detection module 122) trained on scan parameters (pulse sequence commands or control commands 330), scan conditions and results of calibration measurements. Since the images itself are not used for training, personal data is not directly touched. As a result of this AI consistency check, the user could, for example, be asked to review her/his input or the data can be correspondingly annotated.

A typical source of non-image-based data is the logfile of the scanner, which is typically used to store scan settings, results of calibration measurements, error messages and other logging from the scanner hardware in a time resolved manner. Consequently, this logfile data can, e.g., be used to analyze the proper function of the scanner provided, that the input from the user reflects the actual scan setting.

Data consistency strongly depends on user input, since many parameters are currently not independently measured, e.g. anatomy to be imaged, patient orientation, patient position, and etc. Additionally, also other data sources might provide incorrect data, e.g. due to a miscalibrated sensor (table position, ...). As a consequence, a wrong conclusion might be drawn based on the available data. It is proposed to train a machine learning model on the available data, in particular logfile data, which checks the data for consistency.

The scan parameters, the patient "loading" and the results of preparation measurements are typically interrelated, which also means, that there is a degree of redundancy in the data, which can be used to identify incorrect user input or potentially defect sensors. Using the AI model, the user input is predicted based on the available logfile data and in case of discrepancy the user is e.g. asked to confirm the input or the input is tagged. For the proposed approach it is not necessary to analyze the acquired images themselves, so it is not using sensitive data for its purpose.

The B0 and B1 shim settings can be predicted quite accurately based on the information available in the logfile. Already in this context it was found, that some information in the logfile was not sound, e.g., measurements, that had all characteristics of abdominal scans, but were classified as "brain". As a consequence, the prediction model (based on a random forest regressor) can be "turned around" and the anatomy can be predicted based on the calibration results and other information available in the logfile (coils connected, table position, patient orientation, scan parameters, VSWR,...), which allowed to filter out data, that is not correct. Examples may, for example, implement AI models for different features in the logfile (mainly those depending on user input or on sensors which have a chance to be not correctly calibrated). These models can be based on standard machine learning architectures like neural nets, random forest regressors, convolutional networks, etc. If the prediction result does not correspond to the actual feature (potentially including the exceeding of a certain probability limit) the user could be asked to reconfirm the entry or the corresponding sensor is marked as defect. Additionally, the features can be tagged in the logfile accordingly, so that in later data processing either the "correct" predicted value is used or the data is discarded.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: trained outlier detection module
- 124: configuration data
- 126: sensor data
- 128: identification of one or more inconsistent data elements
- 130: signal
- 200: receive the configuration data;
- 202: receive the sensor data from the tomographic medical imaging system;
- 204: input the configuration data and the sensor data into the trained outlier detection module
- 206: provide a signal descriptive of the one or more inconsistent data elements if the identification of the one or more inconsistent data elements is received from the trained outlier detection module
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio-frequency coil
- 316: transceiver
- 318: subject
- 320: subject support
- 330: pulse sequence commands (control commands)
- 332: weight estimation module (trained artifical intelligence module)
- 334: Corrected data
- 336: Modified configuration data
- 338: Modified pulse sequence commands (modified control commands)
- 340: k-space data (tomographic medical imaging data)
- 342: magnetic resonance image (medical image)
- 344: medical Image file (DICOM file)
- 400: receive corrected data in response to providing the signal
- 402: provide modified configuration data by modifying the configuration data using the corrected data
- 404: modify the control commands with the modified configuration data
- 406: control the tomographic medical imaging system with control commands to acquire tomographic medical imaging data
- 408: reconstruct a medical image from the tomographic medical imaging data
- 410: write the medical image, the modified configuration data, and the sensor data to a medical image file
- 500: user interface
- 502: warning
- 504: user entered subject weight
- 506: estimated subject weight (suggested value)
- 508: ignore control button
- 510: instructions
- 512: data entry box
- 514: accept control button

## Claims

1. A medical system (100, 300) comprising:
- a memory (120) storing machine executable instructions (120) and a trained outlier detection module (122), wherein the trained outlier detection module is configured to output an identification of one or more inconsistent data elements (128) in response to inputting configuration data (124) and sensor data (126) from a tomographic medical imaging system (302) as input, wherein the configuration data is descriptive of a configuration of the tomographic medical imaging system to acquire tomographic medical imaging data (340) of a subject (318);
- a computational system (104), wherein execution of the machine executable instructions causes the computational system to:
- receive (200) the configuration data;
- receive (202) the sensor data from the tomographic medical imaging system;
- input (204) the configuration data and the sensor data into the trained outlier detection module; and
- provide (206) a signal (130) descriptive of the one or more inconsistent data elements if the identification of the one or more inconsistent data elements is received from the trained outlier detection module.

2. The medical system of claim 1, wherein execution of the machine executable instructions further causes the computational system to:
- receive (400) corrected data (334) in response to providing the signal, and
- provide (402) modified configuration (336) data by modifying the configuration data using the corrected data.

3. The medical system of claim 2, wherein the medical system further comprises the tomographic medical imaging system, wherein execution of the machine executable instructions further causes the computational system to:
- control (406) the tomographic medical imaging system with control commands (330) to acquire tomographic medical imaging data;
- reconstruct (408) a medical image (342) from the tomographic medical imaging data; and
- write (410) the medical image, the modified configuration data, and the sensor data to a medical image file (344).

4. The medical system of claim 3, wherein execution of the machine executable instructions further causes the computational system to write at least a portion of the signal to the medical image file.

5. The medical system of claim 3 or 4, wherein execution of the machine executable instructions further causes the computational system to modify (404) the control commands with the modified configuration data.

6. The medical system of any one of claim 3, 4, or 5, wherein the tomographic medical imaging system is any one of the following: a magnetic resonance imaging system (302), a computed tomography system, a single photon emission tomography system, a positron emission tomography system; and combinations thereof.

7. The medical system of any one of claims 3 to 5, wherein the tomographic medical imaging system comprises a magnetic resonance imaging system (302), wherein the memory further contains a weight estimation module (322) configured to output an estimated subject (504) weight in response to inputting the configuration data and the sensor data, wherein the configuration data comprises a user entered subject weight (504), wherein execution of the machine executable instructions causes the processor to:
- receive the estimated subject weight by inputting the configuration data and the sensor data into the weight estimation module if the identification of the one or more inconsistent data elements comprises the user entered subject weight; and
- receive a replacement subject weight selected from one of: the estimated subject weight and the user entered subject weight.

8. The medical system of claim 7, wherein the control commands are pulse sequence commands to control the magnetic resonance imaging system to acquire k-space data (340) from an imaging zone (308) at least partially containing the subject, wherein the k-space data is the tomographic medical imaging data, wherein the pulse sequence commands comprise a radio frequency power level setting, wherein execution of the machine executable instructions further causes the computational system to calculate the radio frequency power level setting using the replacement subject weight.

9. The medical system of any one of claims 2 through 8, wherein the corrected data is received from a user interface (500) or from a trained artificial intelligence module (332).

10. The medical system of claim 9, wherein the user interface is configured to display one or more suggested values (506) for the corrected data.

11. The medical system of any one of the preceding claims, wherein the trained outlier detection module is any one of the following: a fully connection neural network and a random forest regression module.

12. The medical system of any one of the preceding claims, wherein the configuration data comprises any one of the following: data used to configure and/or modify a magnetic resonance imaging scan or acquisition, calibration data, include shim currents, a resonance frequency in the imaging zone, metadata descriptive of the subject, a position of the subject, an orientation of the subject, a scan geometry of the subject, modifications made to the pulse sequence commands, a user entered subject weight, an anatomy to be imaged, a logfile of the medical system, error messages of the medical system, B0 shim settings, B1 shim settings, and combinations thereof.

13. The medical system of any one of the preceding claims, wherein the sensor data comprises any one of the following: a position of a subject support, a measurement of the ratios of reflected powers in a magnetic resonance imaging system, an impedance network position, a magnetic resonance coil impedance measurement, an X-ray tube voltage measurement, an X-ray tube current measurement, and combinations thereof.

14. A method of operating a medical system (100, 300) comprising:
- receiving (200) configuration data (124), wherein the configuration data is descriptive of a configuration of a tomographic medical imaging system (302) to acquire tomographic medical imaging data (340) of a subject (318);
- receive (202) the sensor data from the tomographic medical imaging system;
- inputting (204) the configuration data and the sensor data into a trained outlier detection module (122), wherein the trained outlier detection module is configured to output an identification of one or more inconsistent data elements (128) in response to inputting configuration data and sensor data as input; and
- providing (206) a signal (130) descriptive of the one or more inconsistent data elements if the identification of the one or more inconsistent data elements is received from the trained outlier detection module.

15. A computer program comprising machine executable instructions (120) and a trained outlier detection module (122), wherein the machine executable instructions and the trained outlier detection module are configured for execution by a computational system (104), wherein the trained outlier detection module is configured to output an identification of one or more inconsistent data elements (128) in response to inputting configuration data (124) and sensor data (126) from a tomographic medical imaging system (100, 300) as input, wherein the configuration data is descriptive of a configuration of the tomographic medical imaging system to acquire tomographic medical imaging data (340) of a subject (318);
wherein execution of the machine executable instructions causes the computational system to:
- receive (200) the configuration data;
- receive (202) the sensor data from the tomographic medical imaging system;
- input (204) the configuration data and the sensor data into the trained outlier detection module; and
- provide (206) a signal (130) descriptive of the one or more inconsistent data elements if the identification of the one or more inconsistent data elements is received from the trained outlier detection module.
